Europäisches Patentamt

European Patent Office

Office européen des brevets

(19)

(11) Publication number: **0 443 267 A1**

# EUROPEAN PATENT APPLICATION

(12)

(21) Application number: 90313998.8

(22) Date of filing: 20.12.90

(51) Int. Cl.5: **A61B 5/0285**

(30) Priority: 23.02.90 US 484687

(43) Date of publication of application:
28.08.91 Bulletin 91/35

(84) Designated Contracting States:
AT BE CH DE DK ES FR GB GR IT LI LU NL SE

(71) Applicant: SENTINEL MONITORING, INC.
6530 Corporate Drive
Indianapolis, Indiana 46278(US)

(72) Inventor: Smith, Mark Wesley
46 Scottsville-Henrietta Road
Scottsville, New York 14546(US)

(74) Representative: W.P. THOMPSON & CO.
Eastcheap House Central Approach
Letchworth, Hertfordshire SG6 3DS(GB)

(54) Method and apparatus for continuous non-invasive blood pressure monitoring.

(57) Pulse transit time is measured for providing a measure of blood pressure substantially continuously, non-invasively, and non-reactively. Transit time is measured by plethysmographic, electrocardiographic and/or phonocardiographic techniques, and compensation is made for elastic properties of vasculature by means of plethysmographic measurement. Pulse transit time is a measure of pulse wave velocity.

FIG.1

EP 0 443 267 A1

## METHOD AND APPARATUS FOR CONTINUOUS NON-INVASIVE BLOOD PRESSURE MONITORING

The present invention relates to using pulse wave parameters for continuous, non-invasive blood pressure (CNIBP) monitoring, thereby obviating ill effects on the comfort and well-being of the subject whose blood pressure is being measured.

U.S. Patent No. 2,658,505 to Sheer discloses an arterial pulse wave velocity meter, and proposes to calibrate it in terms of blood pressure, to use it to indicate blood pressure and deviations in blood pressure, and to do so continuously and for hours, without difficulty and without discomfort to the patient.

U.S. Patent No. 2,875,750 to Boucke et al proposes measuring blood volume changes due to the arterial or venous pulse, and combining such measurements with an independent measure of systolic or diastolic blood pressure for continuously indicating both systolic and diastolic blood pressure without danger of injury or discomfort to the patient.

U.S. Patent No. 2,944,542 to Barnett et al proposes using photo or impedance plethysmography in measuring pulse wave velocity, and in measuring changes in blood pressure.

U.S. Patent No. 3,090,377 to Salisbury et al proposes measuring the transit time of an arterial pressure pulse, and empirically interpreting it as a measure of diastolic or systolic pressure.

U.S. Patent No. 3,095,872 to Tolles proposes measuring modulation of the arterial pulse wave by injecting a higher frequency wave into the blood flow in an artery. The modulation is interpreted as blood pressure variation, and is indicated as blood pressure by an indicator calibrated in accordance with blood pressure as determined by conventional techniques.

U.S. Patent No. 3, 132,643 to Baum et al proposes measuring "average pressure or essentially the difference between systolic and diastolic pressures" as functions of elapsed times beginning with an electrical cardiac signal and ending with pulse signals.

U.S. Patent No. 3,412,729 to Smith, Jr. proposes measuring pulse pressure photoplethysmographically, and combining such measurement with a measure of systolic pressure in order to get diastolic pressure.

U.S. Patent No. 4,030,485 to Warner proposes measuring pulse pressure photoplethysmographically, and combining such measurement with a measure of mean pressure in order to get systolic pressure.

U.S. Patent No. 4,245,648 to Trimmer et al proposes measuring the rise and transit time of pulse pressure waves, computing systolic pressure and diastolic pressure therefrom, and calibrating these results against a conventional cuff-type sphygmomanometer.

U.S. Patent No. 4,425,920 to Geddes et al proposes measuring diastolic pressure as a function of pulse transit time, and using a microprocessor to relate that measure to a set point, for the purpose of medicating a subject whose blood pressure is to be controlled.

More recently, U.S. Patents Nos. 4,807,638 and 4,869,262 to Sramek and to Orr et al respectively, relate to the present subject matter. Sramek proposes to monitor mean arterial blood pressure as a function of arterial pulse propagation delay determined from bioimpedance measurements. Orr et al propose to measure diastolic blood pressure as a function of heart rate and blood pressure pulse transit time determined by sensing emission of R-waves.

Carruthers et al, "Validation of a New, Inexpensive, Non-Invasive Minaturized Blood-pressure Monitor", Journal of Ambulatory Monitoring, 1988, Volume 1, No. 2, pp. 163-170, appears to relate to a commercial version of the subject matter of the Orr et al patent.

It will be noted that the foregoing prior art refers sometimes to transit time and sometimes to velocity. These terms are functionally equivalent for present purposes, which do not include, as an end, providing a measure of transit time or velocity, as such. That is to say, the end measurement to be obtained is pressure (mean, diastolic, systolic and/or pulse), and the application of particular means adapted to this end can be explained in terms of either transit time or velocity.

It should be noted that the fundamental entity is transit time, i.e. the velocity is given in essence by transit time, inasmuch as the path taken by the pulse wave is the same in either case. Therefore, hereinafter there is used the prior art convention of referring either to transit time or velocity. Various electrical and hydraulic events occur in the cardiovascular system, and from them one can determine time markers signalling the beginning and ending of the time interval which is to be taken as transit time for the pulse wave. This time interval ideally is the time it takes for the aortic pressure wave to go from one point to another in the vasculature. Thus, when the aortic valve opens, that event can be detected and signals the aortic pressure minimum. A little before the aortic valves open, the R part of the QRS complex occurs, and that also can be detected. The Q point can also be used as such a marker.

Further on in the vasculature, the pulse pressure occurs, and cardiovasculature correlates of pulse pressure can be detected in many known ways, for determining fiducial points. In particular, change in arterial blood volume may be measured in a selected portion of microvasculature perfused by arterial blood. In the prior art, such measurement is known as plethysmography and has been carried out in terms of change in impedance, optical density, flow, etc. of the blood in the selected portion of tissue.

Using various combinations of the foregoing events and changes, in the past one has been able to measure pulse wave velocity, generally in terms of the time it takes for the pulse wave to traverse some predetermined arterial path in the vasculature. As is well-known, this velocity can be taken as a measure of diastolic pressure, or, alternatively, of mean effective pressure.

The amplitude of the pulse wave, or the area under the pulse wave, as measured by deflection of the artery wall, or by pulse-caused blood volume changes at perfused tissue sites of the subject, and so on, is taken as measure of pulse pressure, that is to say, the difference between systolic and diastolic blood pressure.

Having obtained pulse velocity and pulse pressure amplitude measurements, one has inferred a disatolic pressure value from the former, and has added the corresponding value of pulse pressure amplitude thereto to get systolic pressure. On living organisms, these measurements are made substantially non-invasively and non-reactively by instruments which contact the external envelope of the organism without penetration and with minimal force or other untoward disturbance of the physiology of the organism.

In practice, the measurements described above are supplemented in various ways in order to improve accuracy, etc. Thus, the instruments which derive systolic and diastolic pressure values from those measures are calibrated periodically against pressure values obtained independently, say by an occlusive cuff system.

In a live human being or other organism having a heart, such heart periodically creates blood pressure pulses in the arterial vasculature of the organism. Thus, when the heart "beats", a ventricle thereof contracts and increases the volumetric rate of flow of blood in the vasculature. At the same time, the contraction force creates a pulse of hydrostatic pressure which propagates as a wave through the blood-filled vasculature. The front of this wave travels at a velocity which is much higher than the velocity at which blood itself flows in the vasculature.

As is known, the pulse pressure wave velocity depends both on the elasticity of the vasculature and on the blood pressure in the vasculature, so according to this invention, plethysmographic effects are measured in tissue of the organism. Such tissue is that in which the vasculature is embedded and perfuses the tissue with arterial blood via a tree of arteries, arterioles and capillaries.

In particular, the optical density of a given portion of said tissue is substantially continuously measured. The amount of blood in said given portion of said tissue fluctuates in response to the pressure pulses created by ventricular contraction. The blood in said portion is primarily that of the vascular bed in said given portion of said tissue, and so is both venous and arterial. As is well known, the venous outflow of the capillary portion of the vascular bed is substantially constant, and non-pulsatile, as compared to the arterial inflow. So, all else being equal, fluctuation in optical density of the said given portion of said tissue quantitatively corresponds to fluctuation in arterial blood volume and, therefore, to the pressure pulse amplitude.

As a first approximation, and for some length of time, the vasculature of any given individual organism can be regarded as a constant, at least insofar as the elasticity thereof is concerned, and, so, for that individual, during that length of time, measuring pressure pulse wave velocity as such can usefully be thought to provide a measure of its blood pressure.

However, the vasculature of such individual organisms is frequently not a constant, i.e. the organism breathes, may receive therapy, is subject to physiological/psychological stress, and so on. Hence, it becomes likely that optical density of the said given portion will, for a given organism, represent factors other than just the effect of pulse pressure in a vasculature of given, fixed elasticity.

According to the invention, the effect of said factors is obviated by providing an optical density measurement which distinguishes between the plethysmographic effect of pulse pressure and plethysmographic effects due to the aforesaid other factors.

In one embodiment of the invention, two photoplethysmographic devices of known construction are used. One device has a probe which has a light source which illuminates a first given portion of tissue with a beam of infrared (IR) light having a wavelength so chosen that the beam will be relatively insensitive to the oxygen present in the blood which perfuses said portion. The other device similarly illuminates a second given portion of said tissue but at a location such that the length of path, through the vasculature which perfuses said portions with blood, and between the first given portion and the heart, is different from the length of path through said vasculature, and between the second given portion and the heart.

Each probe has an IR light sensor producing a signal whose amplitude is proportional to the intensity of IR light received from the corresponding said given portion. Also, the probes are arranged such that their sensors receive substantially only light from said given portions, i.e. not directly from their light sources, and preferably not from other sources in their immediate environment.

In the short run, as the given portions of tissue do not change their dermal, fleshy and other structural properties, so the fluctuations of the sensor signals represent pulses of the total blood volume in the given portions.

In accordance with the invention, the pulsations due to blood volume change are separated from the total signal which contains a much larger, but much more slowly varying component due to such structural properties as mentioned above. The pulsations due to blood volume change alone are a measure of pulse pressure, that is to say, of the difference between systolic and diastolic blood pressure in the vasculature.

According to the invention, also, the blood volume pulsations are used as markers by which a measure may be made of the transit time of a wave front of a pulse pressure wave created by ventricular contraction, in a path of predetermined length of vasculature.

Furthermore, according to the invention, after separating pulsations due to blood volume change from the total photoplethysmograph signal, the amplitude of the remaining signal reflects venous blood, non-blood tissue, and the effects of possible iatrogenic, psychogenic, respiratory and/or other influences. Inasmuch as these influences, if not constant, or absent, would introduce variable error into a measure of blood pressure, under the assumption that the organisms vasculature was constant, such measure is therefore modified by the above-described signal remaining after the pulsations of blood volume have been separated from the total probe signal.

As a result, once calibrated, for a given subject, on a particular occasion, that calibration may be relied on for some while despite the influences of stress, medication, breathing, and so on. For longer periods of time the measurement of blood pressure according to the invention needs also to be calibrated from time to time against some standard. Such calibration may be performed at intervals long enough that there need be substantially no concern of the effect on the subject, due to the instrument providing the standard, because in between such standardisings, the invention can otherwise be relied on for continuous monitoring of blood pressure with no ill effect on the subject.

A preferred embodiment of the invention will now be described by way of example and with reference to the accompanying drawings, in which:

Figure 1 is a block diagram of the CNIBP monitoring system according to the present invention; and

Figure 2 is a diagram illustrating cardiac events determinable from phonocardiographic and electrocardiographic measurements, aortic and left ventricular pressure measurements, and left ventricular volume measurements.

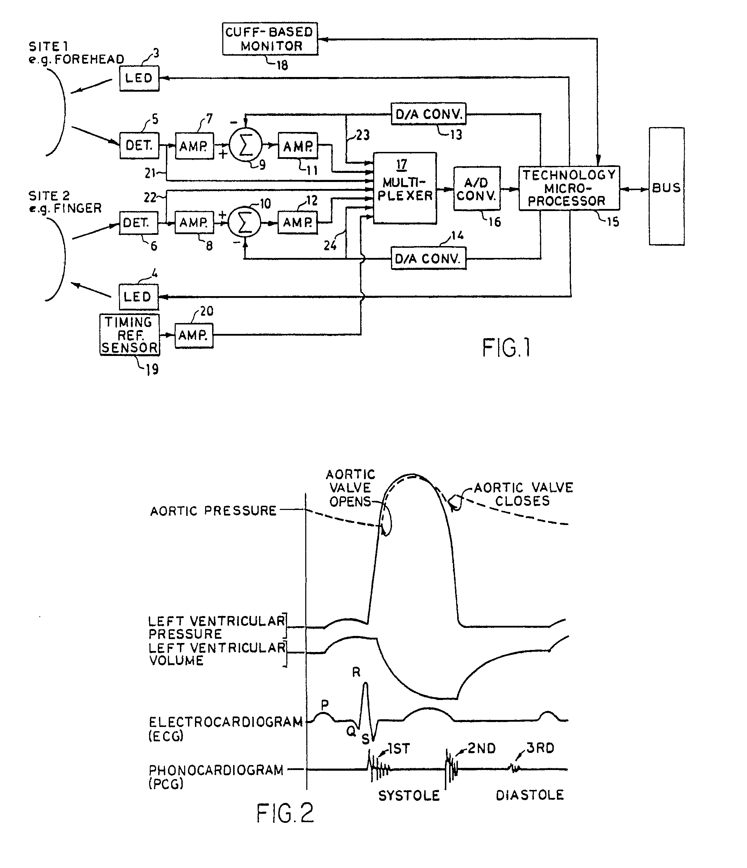

In Figure 1, suitable sites 1 and 2, e.g. a living being's forehead and finger, are illuminated by LEDs 3 and 4 respectively, and respective detectors 5 and 6 are illuminated by light returning from sites 1 and 2. The LED light outputs are fixed in amplitude, whereas the amplitude of light returning from sites 1 and 2 is modulated by the vascular bed at sites 1 and 2. Preferably, the LEDs and detectors are incorporated in probe structure (not shown) which isolates the illuminated sites and the detectors from ambient light. Also, such probe structure isolates the detectors from all the LED light save that which comes from the illuminated sites.

The detectors 5 and 6 convert the light they receive into an electrical signal, in this case a direct current. The amplitude of this direct current is proportional to the amplitude of the light received by the detectors. The detectors feed such signal to transconductance amplifiers 7 and 8 respectively, each fitted out with conventional circuitry (not shown in Figure 1) which makes them convert the detector current at their inputs into voltages at their outputs varying in amplitude in proportion to the amplitudes of those currents at their inputs.

The output voltages of amplifiers 7 and 8 are next applied to respective summing junctions 9 and 10 along with the respective output voltages of digital to analog (D/A) converters 13 and 14. The D/A converter output voltages result from application to the converters of digital voltages from outputs of a technology microprocessor module 15, which has processed signals from an analog to digital (A/D) converter 16 receiving analog voltages from a multiplexer 17, and converting the analog voltages to digital form. Multiplexer 17 receives the output voltages of amplifiers 11 and 12, and presents these analog voltages, and others, one at a time, to the A/D converter 16.

A cuff-based pressure monitor 18 provides for applying a digital voltage representing an independently, and generally intermittently, measured blood pressure of the being whose forehead and finger (or equivalent portions of perfused tissue) provide sites 1 and 2.

A timing reference sensor 19 provides a voltage representing the occurrence of some event in the cardiovascular system, which voltage, after amplification by an amplifier 20, is sampled by multiplexer 17 and passed on thereby to A/D converter 16. Of course, sensor 19 is not necessary if, as here, two LED-detector sets are used.

The technology microprocessor module 15 is so termed because it incorporates the pressure measuring algorithm for processing the information available through multiplexer 17 and from monitor 18, by virtue of the corresponding technology represented by the particular hardware and concept of measurement. It is to be noted that it is not the nature of the hardware alone which determines what the technology is. Thus, in oximetry, whereas only one of sites 1 and 2 need be used, both LEDs would be necessary, so nothing need be changed by the spectral character of one LED and the algorithm in the microprocessor, which would incorporate the concept of oximetry instead of blood pressure measurement.

In the present case, each of the LEDs 3 and 4 can be the same infrared LED used in EP-A-0341059. Likewise, each of the detectors 5 and 6 and transconductance amplifiers 7 and 8 can be chosen to be the same as the corresponding detector and current to voltage converters of EP-A-0341059. However, here the concern is not with frequency multiplexing red and infrared signals, for a two signal channel arrangement, but with time multiplexing.

According to the invention, sites 1 and 2 provide plethysmographic information both as to pulse volume, that is the amount by which the volume of blood perfusing one of the sites changes due to the pulse wave, and as to pulse wave velocity, as given by pulse transit time, that is the length of time it takes for a given pulse wave front at the site nearer the heart to show up at the more distal site.

Alternatively, according to the invention, the plethysmographic information obtained could be relied on simply for pulse volume. In that case, only one site, and hence one LED-detector set, etc., would be required, but then it would be necessary to utilise electrocardiographic or phonocardiographic information, a capability represented by timing reference sensor 19, in order to get a measure of pulse transit time or pulse wave velocity. The single plethysmographic site would act as the distal site with respect to the heart, which would then act as the nearer site, so to speak. Again, ultrasonic flow detecting arrangements can provide fiducial signals.

The function of the summing junctions 9 and 10, according to the invention, is to keep the slowly varying component of the output voltage of the amplifiers 7 and 8 out of amplifiers 11 and 12. As disclosed in EP-A-0341059, at this point, most of the information carried by the frequency modulated carrier waves represents the same information as is carried by the aforesaid slowly-varying component, but which is eventually removed after demodulation of the carrier waves. According to the present invention, feedback loops, which extend via the multiplexer 17, A/D converter 16, microprocessor 15, D/A converters 13 and 14, to respective summing junctions 9 and 10, prevent the aforesaid slowly-varying component from being applied to the inputs of amplifiers 11 and 12. Consequently, the amplifiers 11 and 12 can be constructed to have relatively high voltage gain because they will receive from the summing junctions 9 and 10 just the relatively small AC component remaining after summation at the summing junctions 9 and 10.

According to the invention however, the feedback signals are software-created in microprocessor 15 from the outputs of detectors 5 and 6. Thus, in addition to the expected unnumbered connections depicted in Figure 1 as interconnecting variously the thus far described components 3 to 20, respective numbered connections 21 and 22 are shown between the outputs of detectors 5 and 6 and multiplexer 17. In addition, between the multiplexer 17 and the outputs of D/A converters 13 and 14 are respective numbered connections 23 and 24.

As disclosed in EP-A-0341059, the AC part of the photoplethysmographic signal is extremely small as compared to the total photoplethysmographic signal. Further, the total signal also inevitably contains a noise portion due to residual ambient light among other things, and normally not much different in magnitude from the AC part. According to the present invention, connections 21 and 22 provide for applying the total photoplethysmographic signals to the microprocessor module 15. The microprocessor of module 15 is programmed to convert those signals into ones of slightly lesser amplitude, and to feed them back via the respective D/A converters 13 and 14 to summing junctions 9 and 10.

It will be noted that amplifying the total photoplethysmographic signal and providing A/D and D/A conversion with resolutions adequate for handling the small AC portion as part of the total signal poses design difficulties and/or unfavourable costs. However, as a result of the present invention, one can utilise a 12 bit A/D converter for A/D converter 6 and 8 bit D/A converters for D/A converters 13 and 14 and, as well, relatively high AC gain in amplifiers 11 and 12, none of which would be feasible if the AC signal had to be processed as part of the total signal.

The multiplexer 17, operating at a kiloHertz rate, feeds the site 1 and 2 signals, one at a time, to the microprocessor of module 15, and they are used then by the microprocessor of module 15 to create and to apply, again one at a time, to the corresponding summing junctions, signals having magnitudes which are, say, 95% of the site signals from which they resulted. These 95% signals are subtracted at the summing junctions from the then-current site signals from amplifiers 7 and 8. This leaves 5% signals for comfortable amplification by amplifiers 11 and 12 at a gain of, say, 25. That is, as each 95% signal comes back to a summing junction, it finds there the current signal from the site which originally gave rise to the 95% signal. The sampling rate of the multiplexer is high enough that the site signals will not change so much that 95% of it will be significantly different from the 95% signal from the microprocessor. As one skilled in the art will recognise, signals from the detectors can be interleaved, so to speak, with "dark" signals and the like, for the usual purposes.

All signals are sampled by the multiplexer 17, one after the other, and eventually the ultimate pressure measurement is output by module 15 to BUS, for distribution to suitable conventional means (not shown) for indicating, recording, alarming, controlling, or the like.

A basic procedure for the practice of the invention is as follows:

For a plurality of heart beats and preferably on each one of consecutive beats throughout a time interval, the length of which will depend on how often it is deemed necessary to recalibrate against a conventional blood pressure monitor, pulse transit time is measured, say between sites 1 and 2. On the same beat, and at one of the sites 1 and 2, the total plethysmographic signal is measured, say that sensed by detector 5 at site 1.

In the microprocessor module 15, suitable software then estimates diastolic pressure as a value proportional to 1/PTT, where PTT stands for pulse transit time. The precise numerical relationship is initially determined empirically.

Also, the software estimates pulse pressure, PP, as a function of diastolic pressure, PD, and pulse volume, VP. In turn, VP is determined by splitting the total plethysmographic signal into its AC and DC components, as in the oximeter in EP-A-0341059, and taking the ratio of the AC component to the DC component (corrected for the effect bloodless tissue would have on the photoplethysmographic signal).

In particular:

(2) $PP = PD(expKVP-1)$, where K is a calibration constant, empirically determined, which is redetermined each time that the system is recalibrated against cuff-basedmeasurements. N.B. All empirical and recalibration operations are obtained from AC and DC signal components determined just before inflating the cuff.

In particular:

(2) $K = (1n(PSC/PDC))/VP$, where PDC and PSC are diastolic and systolic calibrating pressures as measured by the cuff-based monitor.

In Figure 2, typical graphs of aortic pressure, left ventricular pressure, left ventricular volume, electrocardiac activity, and phonocardiac activity are shown for one arterial pulsation of a single subject. As will be seen from the graphs, there are more possibilities for fiducial points than have been referred to above. Some, like ECG, do not exactly correlate with the pulsation, whereas others do. Thus, the R wave of the ECG slightly precedes expulsion of blood from the left ventricle, but on the other hand the second heartsound of the PCG begins on closing of the aortic valve. N.B. PCG is shown here in the form it has when taken directly at the heart, but there will be delays before second sound will be detectable at sites distal to the heart. However, one site would be more distal than the other so the difference between the delays will give the transit time between sites, independently of the time taken for the pulse wave to get from the heart to the closer site.

## Claims

1. A method of measuring blood pressure in a living being which comprises the steps of:

    (a) providing a first measure in the form of a substantially non-invasive and non-reactive measure of pulse wave velocity in the arterial vasculature of said living being;

    (b) providing a second measure in the form of a substantially non-invasive and non-reactive measure of pulse wave amplitude or area in said vasculature, said second measure being made by substantially non-invasively and non-reactively measuring pulsatile blood volume change, said pulsative blood volume change being due to the pulse of said wave, and in a portion of said being's arterially perfused tissue; and

    (c) processing said first and second measures to provide a measure of blood pressure therefrom.

2. A method as claimed in claim 1, characterised in that said first measure is obtained by optical plethysmography.

3. A method as claimed in claim 1, characterised in that said second measure is obtained by optical plethysmography.

4. A method as claimed in claim 1, characterised in that both of said measures are obtained by optical plethysmography.

5. A method as claimed in any preceding claim, characterised by the step of providing a third measure for compensation of said second measure, said third measure being made by substantially non-invasively and non-reactively measuring variations of total blood volume change in said portion of tissue.

6. A method as claimed in claim 5, characterised in that all of said measures are obtained by optical plethysmography.

7. Apparatus for measuring blood pressure in vasculature containing blood which is caused to flow therethrough for perfusing, with blood, tissue in which said vasculature is embedded, and wherein pulsation of said blood pressure is caused by periodic contraction of a ventricle supplying said vasculature with said blood, said apparatus comprising, in combination,

(a) first means for producing a first signal, said first signal representing the velocity of propagation in said vasculature of said pulsation;
(b) second means for producing a second signal, said second signal representing the amplitude or area in said pulsation; and
(c) third means receiving said first and second signals and arranged to derive therefrom a measure of said blood pressure.

8. Apparatus according to claim 7, characterised in that said first means is effectively responsive to said contraction for causing said first signal to represent the velocity at which said pulsation is propagated along a predetermined path in said vasculature, beginning substantially with said contraction, or substantially at the site of said contraction.

9. Apparatus according to claim 7, characterised in that said first means is responsive to an electrical signal corresponding to said contraction for causing said first signal to represent the velocity at which said pulsation is propagated along a predetermined path in said vasculature, beginning substantially with said contraction, or substantially at the site of said contraction.

10. Apparatus according to claim 7, characterised in that said first means is responsive to an acoustic signal corresponding to said contraction causing said first signal to represent the velocity at which said pulsation is propagated in a predetermined time interval, or along a predetermined path in said vasculature, beginning substantially with said contraction, or substantially at the site of said contraction.

11. Apparatus according to claim 7, characterised in that said first means is responsive to volumetric change in said tissue for causing said first signal to represent the velocity at which said pulsation is propagated in a predetermined time interval defined by said volumetric change.

12. Apparatus according to claim 11, characterised in that said second means includes said first means.

13. Apparatus according to claim 7, characterised in that said first means is responsive to volumetric change in spaced portions of said tissue, for causing said first signal to represent the velocity at which said pulsation is propagated between said portions in a time interval defined by said volumetric change.

14. Apparatus according to claim 13, characterised in that said second means includes said first means.

15. Apparatus for providing a measure of blood pressure in the vasculature of a living organism wherein blood flows through said vasculature in substantially periodic pulses and perfuses tissue of said organism, and wherein the total blood volume perfusing a given portion of tissue includes venous blood as well as arterial blood, said apparatus comprising, in combination:

(a) first means for measuring the volume of an arterial blood pulse in said given portion;
(b) second means for measuring the total blood volume in said given portion;
(c) third means for measuring the velocity of arterial blood pulses in said vasculature; and
(d) fourth means connected to said first, second and third means for taking the respective measurements thereof, and deriving therefrom said measure of blood pressure.

16. Apparatus according to claim 15, characterised in that said first means and second means includes a plethysmograph arranged relative to said given portion for providing a volume measurement, said apparatus also including means for causing a measure of arterial blood pulse volume to result from said volume measurement, and for causing to result a measure of the total blood volume in which said arterial blood volume was contained.

17. Apparatus according to claim 16, characterised in that said plethysmograph is of the kind which directs light on said given portion and detects the intensity of light consequently emitted from said portion, the spectral nature of the light directly on said given portion being such that said intensity is a function of the amount of tissue and blood in tissue perfused by the aforesaid blood volume, for thereby achieving a measure of blood pressure which is relatively uninfluenced by oxygenation of the blood in said portion.

18. Apparatus according to claim 16, characterised in that said third means includes both said plethysmograph and a pulse detector means for detecting a pulse of arterial blood in said vasculature, said pulse detector means being constructed and arranged for detecting the said pulse at a point in said vasculature such that the arterial pulse corresponding to said measure of arterial blood pulse volume is detected at a time which is different from the time at which it occurred in said vasculature during said volume measurement.

19. A non-invasive method for continuous measurement of blood pressure values, said method including the steps of:
    (a) providing a measure of blood pressure pulse wave velocity;
    (b) providing a measure of change in total blood volume, said change corresponding to said pulse wave and being in a perfused microvascular region;
    (c) providing a measure of change of arterial volume corresponding to said pulse wave and being in said region; and
    (d) combining said measures so as to provide a measure of blood pressure.

20. A method as claimed in claim 19, characterised in that said measure of blood pressure is scaled with respect to a prior independent measurement of blood pressure.

21. A method as claimed in claim 19, characterised in that the measure of step (a) is derived from step (c), the measure of step (a) also providing a measure of blood pressure.

22. A method as claimed in claim 19, 20 or 21, characterised in that said measure of pulse wave velocity is used to adjust said measure of microvascular blood volume change for providing a measure of arterial pulse volume which is substantially insensitive to local blood flow changes uncorrelated with systemic arterial volume changes.

23. Apparatus for measuring pulsating blood pressure, comprising in combination:
    first means for sensing blood volume in a first portion of blood-perfused tissue and producing a first signal, said first signal representing the magnitude of the corresponding said blood volume;
    second means for sensing blood volume in a second given portion of blood-perfused tissue and producing a second signal, said second signal representing the magnitude of the corresponding said blood volume;
    third means for receiving said first and second signals and in response thereto producing a third signal, said third signal representing the time it takes for a pulsation of said blood pressure to travel between said given portions;
    the said given portions being spaced along vasculature perfusing said portions with blood, and said blood transmitting said blood pressure pulsations to blood in said portions whereby to vary the volume of blood therein in accordance with said blood pressure pulsations; and
    fourth means for receiving said third signal and one of said first and second signals and, in response thereto, producing a fourth signal representing the magnitude of said pulsating blood pressure.

FIG.1

FIG.2

European
Patent Office

**EUROPEAN SEARCH
REPORT**

Application Number

**EP 90 31 3998**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | WO-A-8 503 211 (JOHNSON MATTHEY PUBLIC CO. LTD.)<br>* page 2, line 16 - page 4, line 27; figures 1-3 * | 1-4 | A 61 B 5/0285 |
| A | | 5-7,10, 11,15-19, 23 | |
| Y | WO-A-8 908 424 (VECTRON GESELLSCHAFT)<br>* abstract; page 5, line 17 - page 6, line 16; figure 2 * | 1-4 | |
| A | | 7,19,23 | |
| A | DE-A-2 460 839 (VOLKSWAGENWERK)<br>* page 2, line 3 - page 4, line 4; page 5, line 10 - page 7, line 5; figure * | 1,2,4,7, 19,23 | |
| A | FR-A-1 531 412 (ELECTRONIQUE MARCEL DASSAULT)<br>* page 1, left-hand column, lines 32-39, right-hand column line 16 - page 2, right-hand column, line 4; figures 1,2 * | 1,7,19,23 | |
| A | ELEKTRONIK vol. 29, no. 12, June 1980, pages 49-52, München, DE; K. SINGER et al.: "Die Messung der Pulswellen-Geschwindigkeit beim Menschen"<br>* page 50, left-hand column, lines 27-47; figures 1,2 * | 1,2,4 | TECHNICAL FIELDS SEARCHED (Int. Cl.5)<br><br>A 61 B 5/00 |

The present search report has been drawn up for all claims

| Place of search | Date of completion of search | Examiner |
|---|---|---|
| Berlin | 03 May 91 | WEIHS J.A. |